# EUROPEAN PATENT SPECIFICATION

(11) **EP 4 194 844 B1**
(45) Date of publication and mention of the grant of the patent: **01.07.2026**
(21) Application number: 23153760.6
(22) Date of filing: 28.11.2019
(51) Int. Cl.: G01N 27/22, A61B 5/00, A61F 13/42

(54) **A SENSOR, SYSTEM AND METHOD FOR DETECTING OR SENSING MOISTURE OR WETNESS OF AN ARTICLE**
SENSOR, SYSTEM UND VERFAHREN ZUR DETEKTION ODER MESSUNG VON FEUCHTIGKEIT ODER FEUCHTIGKEIT EINES ARTIKELS
CAPTEUR, SYSTÈME ET PROCÉDÉ DE DÉTECTION OU DE DÉTECTION D'HUMIDITÉ OU D'HUMIDITÉ D'UN ARTICLE

(30) Priority: 28.11.2018 NZ 18748811
(43) Date of publication of application: 14.06.2023
(62) Divisional of application: 19816925.2
(73) Proprietor: Tilkoblede Belgium BVBA, 1702 Dilbeek (BE)
(72) Inventor: VAN DE SANDE, Benoit, 1703 Schepdaal (BE); VAN DE SANDE, Bram, 1703 Schepdaal (BE)
(74) Representative: Mewburn Ellis LLP

(56) References cited:
- WO-A1-2015/184148
- WO-A2-2009/084942
- US-A1- 2014 026 653
- US-A1- 2014 291 677

## Description

### TECHNICAL FIELD

The present disclosure generally relates to a sensor for detecting or sensing moisture or wetness of an article to which the sensor is attached.

### BACKGROUND

Many different types of articles are used in a typical household. During use of these articles they may come into contact with a liquid such as water and or aqueous solutions or mixtures. The presence of the liquid may not be immediately observable, and so timely and adequate actions in response cannot be determined.

The article must then be constantly checked to determine the presence of water.

An example of such an article is an absorbent article, in particular a disposable personal care article, such as a nappy, a diaper, a baby pant, an adult incontinent garment, and the like. Once the nappy has been fitted there is no way to tell whether liquid (such as urine) is present in the nappy.

A further example may be a piece of clothing for example a layer in a multi layered outfit. The outfit may have an external waterproof layer, with multiple under layers to ensure the wearer is kept warm. If the waterproof layer is breached an outer layer of the under layers is exposed to the elements, the outer layer may become wet. Because of the other intermediate layers between the wearer and the outer layer, the breach of the waterproof layer may not be immediately apparent to the wearer leading to further water ingress.

Chemically activated indicators are available which contain various chemical compounds which undergo a chemical reaction when exposed to moisture. These indicators are generally single use and may not indicate the amount of moisture present.

Indicators containing sensors are also available, utilising capacitive, resistive, inductive, optical and sonar sensors.
US 2014/026653 A1 relates to capacitive sensors utilised for gas (including humidity) concentration measurements.
WO 2009/084942 A2 relates to a transducer comprising a planar capacitor for measuring moisture and humidity content.
US 2014/291677 A1 relates to the fabrication of application-specific integrated circuits that incorporate thin film environmental sensors for temperature, pressure and humidity.
WO 2015/184148 A1 relates to a heated capacitor and a method of forming the heated capacitor which removes moisture from a moisture-sensitive insulating layer.

### SUMMARY OF THE INVENTION

The invention is defined by the appended claims.

In some embodiments, the first portion of the second plate is located at or adjacent a first edge of the first plate, and wherein the second portion of the second plate is located at or adjacent a second edge of the first plate.

In some embodiments, the first edge is located adjacent the second edge and/or wherein the first edge is located opposite the second edge.

The first plate is configured to be located, in use, nearer to article to which the sensor is attached than the second plate, or the second plate is configured to be located, in use, nearer to article to which the sensor is attached than the first plate.

In some embodiments the intermediate layer is an insulating layer and a dielectric layer.

In some embodiments, the intermediate layer is a substrate to which the first plate and second plate are attached, preferably a PCB, more preferably a flexible PCB.

In some embodiments, optionally in combination with any one of the previous embodiments, the measurement means are configured to charge a capacitor formed by the first and the second plate and the intermediate dielectric layer, to actively discharge said capacitor, and to measure an electric signal, preferably a voltage, between the first and the second plate after said actively discharging.

In some embodiments, the electrical components are one or more of:
a micro controller
a battery or other energy source
a comparator
an analog to digital converter
an integrated component
a resistor
a transistor
a capacitor
an inductor
a switch
a diode and/or LED
a resonance crystal
an antenna
a transistor
a communications module
a display module.

In some embodiments, the first plate is provided on a first side of the sensor, wherein the first side is configured to be located towards the article in use.

In some embodiments, the second plate is provided on a second side of the sensor, wherein the second side is configured to be located away from the article in use.

In some embodiments comprising a protective layer, the protective layer is provided as an external layer configured to provide protection of the sensor.

In some embodiments, the protective layer is configured to encapsulate the sensor.

In some embodiments, the protective layer is or comprises one or more of: a polymer layer, or paint, or latex paint, or rubber or glass.

In some embodiments, the protective layer is configured to be an electrically insulating material.

In some embodiments, the protective layer is configured to be less than about 25mm in thickness, or between about 5 and about 20 mm in thickness.

In some embodiments, the sensor comprises an attachment portion.

In some embodiments, the attachment portion is provided on a side (optionally a first side) of the sensor.

In some embodiments, the attachment portion is configured to allow for attachment of the sensor to the article.

In some embodiments, the attachment portion allows for connection and disconnection of the sensor to the article.

In some embodiments, the attachment portion comprises one or more of:
a hook and/or loop material
an adhesive
a pin or button
an electrostatic attachment mechanism.

In some embodiments comprising an isolation layer, the isolation layer comprises an insulating layer.

In some embodiments, the insulating layer of the isolation layer is a polymer layer.

In some embodiments, the isolation layer comprises a conducting sheet.

In some embodiments, the isolation layer is configured to isolate the sensor from the external environment.

A technical advantage of the present invention is achieved by measuring the ionisation of the environment around the sensor accurately by using the sensor configuration (having a first and second plate) as described.

A further technical advantage may be achieved as the present arrangement does not require full discharge of the electromechanical cell between measurements as is required with capacitive measurement techniques.

It should be understood that alternative embodiments may comprise any or all combinations of two or more of the parts, elements or features, or applications illustrated, described or referred to in this specification.

### BRIEF DESCRIPTION OF THE DRAWINGS

Fig. 1 shows a bottom view of a sensor.
Fig. 2 shows a top view of a sensor.
Figs. 3 to 5 show cross sections of a sensor.
Fig. 6 shows an example output of a sensor.
Fig. 7 shows top and bottom views of a system including a sensor.
Fig. 8 shows a system attached to an article.
Fig. 9 shows a circuit of the sensor/system.
Fig. 10 shows a flow diagram of a measurement cycle for the system.
Fig. 11 shows a diagrammatic overview of the system.
Fig. 12 shows a graph of measurement readings taken from a sensor at different moisture or wetness levels, and other associated data points.
Fig. 13 shows a flow diagram of a method using 1^{st} and 2^{nd} derivatives.
Fig. 14 shows a series of output graphs (200, 300, 400) from an exemplary sensor wherein series 200 is raw sensor output, and series 300 is a filtered output, and series 400 is a first derivative output.
Fig 15A shows a schematic illustration front view (looking at second side 22) of a sensor, having an array of sub sensors 10.
Fig 15B shows a schematic illustration back to view (looking at first side 21) of a sensor, having an array of sub sensors 10.
Fig 15C shows a schematic cross section view of the sensor of figure 15 A.
Fig 16A shows a schematic illustration of a further alternative sensor 10 configuration.
Fig 16B shows a schematic cross section view of the sensor of Fig. 16A, along line AA.
Fig 16C shows a schematic cross section view of the sensor of Fig. 16A, along line BB.

### DETAILED DESCRIPTION

Disclosed is a sensor, system including the sensor, and method for detecting or sensing moisture or wetness of an article to which the sensor is attached.

The sensor may be configured to measure the presence of any liquid or fluid. For example, in the case where the article is a nappy for a child, the sensor may be configured to measure the presence of urine.

The sensor 10 may be a sensor 10 which has an output which is indicative of the moisture or wetness of an article 28.

In particular, it will be appreciated that the sensor is preferably capable of sensing moisture or wetness of an article without being directly in contact with the moisture or wetness. That is, the sensor is capable of sensing the moisture or wetness within a nappy (for example), when attached to the outside (dry side) of the nappy.

This feature is highly desirable as it allows any one or more of the following advantages:
- ease of adding/removing sensor from article
- ease of access to sensor (particularly useful where sensor module provides visual and/or audible output, or requires other interaction and/or servicing such as charging, re-charging, downloading data etc)
- keep sensor hygienically clean, which makes the apparatus more user-friendly and/or more easily re-usable.

The sensor 10 may be a capacitive type sensor which varies in capacitance depending on the moisture or wetness content of the article 28. For example, an article with a relatively higher moisture content would lead to a relatively higher capacitance of the sensor 10 as compared to an article with a relatively lower moisture content.

In some embodiments, the sensor may be a sensor for which the sensor's charge storage capacity changes depending on the moisture or wetness content of the article 28. For example, an article with a relatively higher moisture content would lead to a relatively higher charge storage capacity of the sensor 10 as compared to an article with a relatively lower moisture content.

In some embodiments the sensor may be an inductive type sensor, and the inductance may vary based on the moisture content of the article.

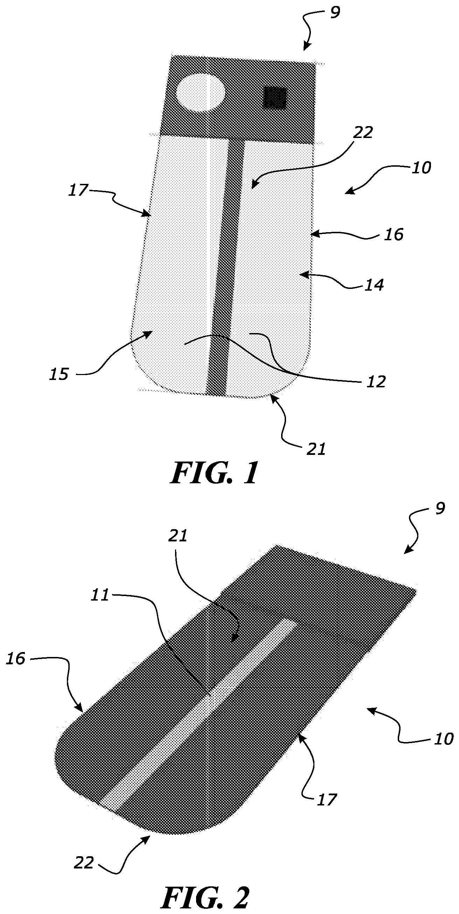

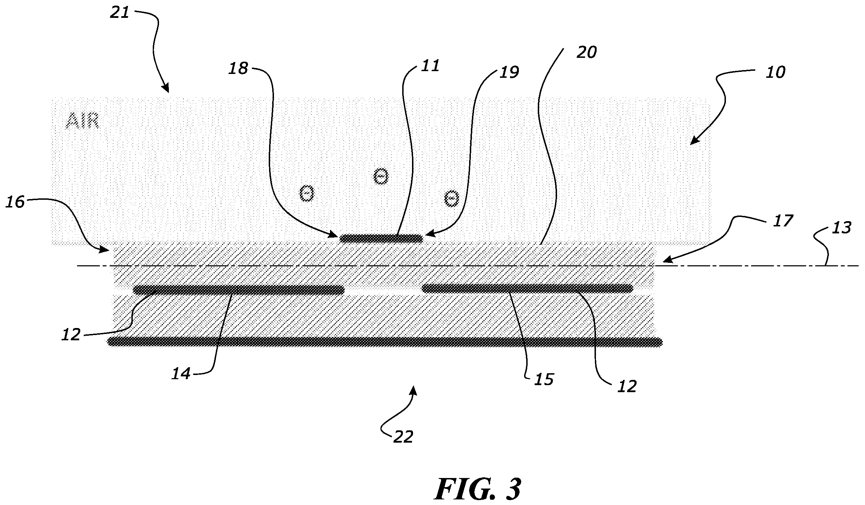

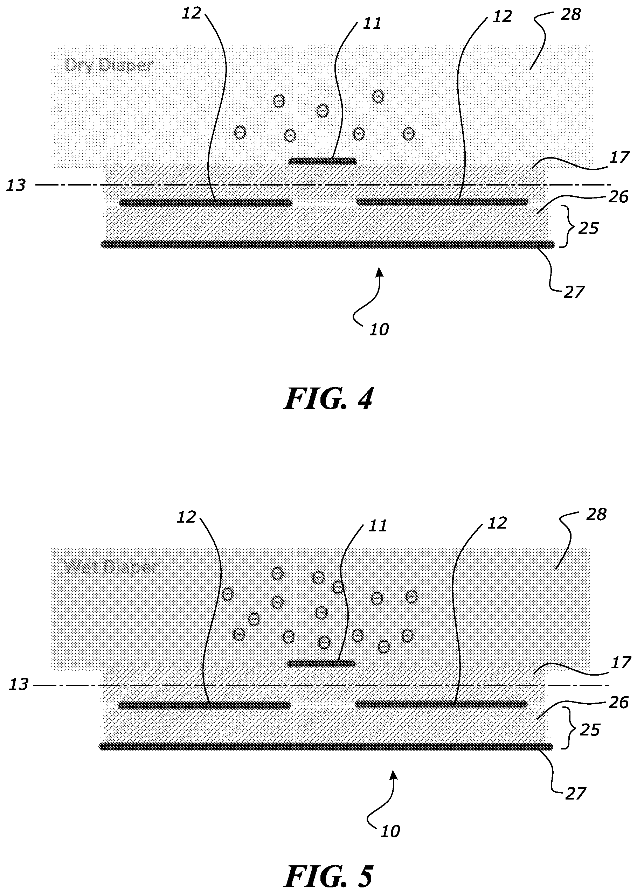

Figs. 1 to 5 show a sensor 10. The sensor comprises a first plate 11 and a second plate 12. The first plate 11 and the second plate 12 are electrically conductive.

The first plate 11 and second plate 12 may be made of a metallic material such as copper, or aluminium, or tin, or lead or any conductive metal, or alloy.

The first plate 11 and second plate 12 may be oriented along and/or substantially parallel to a nominal sensor plane 13. The sensor plane 13 may be oriented, in use, substantially parallel to the article 28 to which the sensor 10 is attached.

The first plate 11 may be located between a first portion 14 of the second plate 12, and a second portion 15 of the second plate 12 (when viewed in plan view).

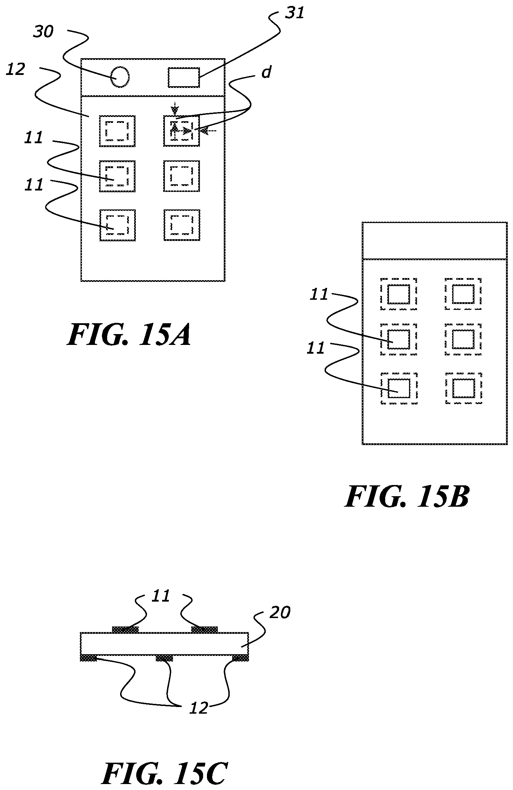

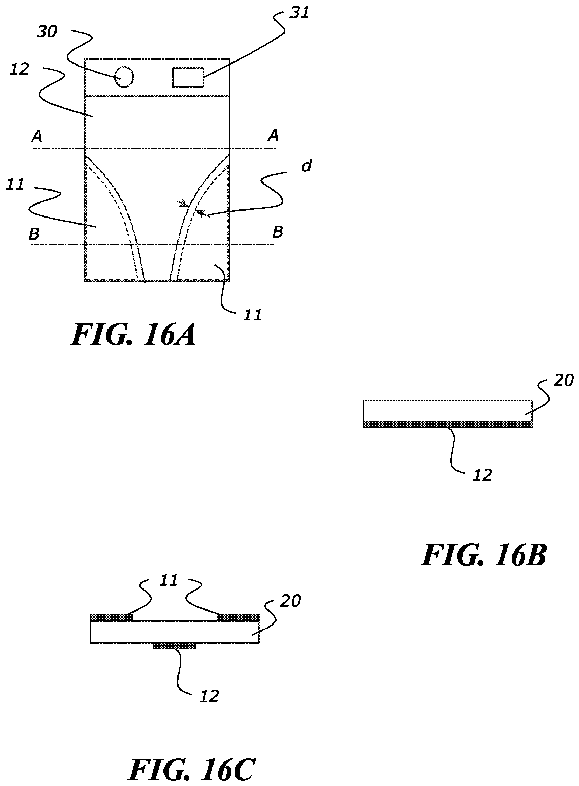

The first portion 14 of the second plate 12 may be located at or adjacent a first edge 18 of the first plate 11, or alternatively near a first edge 18 of the first plate 11 such that no overlap occurs, or such that less than approximately 5% (of the area of the second plate) of overlap occurs when viewed in plan view. Alternatively still, a small gap (equivalent to 'd' in Figs. 15 and 16) may be present.

The second portion 15 of the second plate 12 may be located at or adjacent a second edge 19 of the first plate 11, or alternatively near a second edge 19 of the first plate 11 such that no overlap occurs, or such that less than approximately 5% (of the area of the second plate) of overlap occurs when viewed in plan view. Alternatively still, a small gap (equivalent to 'd' in Figs. 15 and 16) may be present.

The first portion 14 of the second plate 12 may be located at or adjacent a first edge of the sensor, or alternatively near a first edge of the sensor such that no overlap occurs, or such that less than approximately 5% (of the area of the second plate) of overlap occurs when viewed in plan view. Alternatively still, a small gap (equivalent to 'd' in Figs. 15 and 16) may be present.

The second portion 15 of the second plate 12 may be located at or adjacent a second edge of the sensor 10, or alternatively near a second edge of the sensor such that no overlap occurs, or such that less than approximately 5% (of the area of the second plate) of overlap occurs when viewed in plan view. Alternatively still, a small gap (equivalent to 'd' in Figs. 15 and 16) may be present.

The first edge of the sensor 10 may be located adjacent the second edge and/or the first edge of the sensor 10 may be located opposite the second edge.

The first edge 18 of the first plate 11 may be located adjacent the second edge 19 and/or the first edge 18 of the first plate 11 may be located opposite the second edge 19.

Each portion (for example the first portion 14 and the second portion 15) of the second plate 12 may be located on each side of the first plate 11.

The second plate 12 may comprise one or more further portions. The one or more further potions may be located at or adjacent one or more edges of the first plate 11.

The first plate 11 may be or comprise an elongate strip.

The second plate 12 may comprise a pair of elongate strips. Each elongate strip of the pair of elongate strips may be one of the first portion 14 or the second portion 15 of the second plate 12.

In preferred embodiments, when seen in a projection on a plane including the second plate, the shape of the projection of the first plate on the plane is substantially complimentary with the shape of the second plate.

The first plate 11 may be for example a X or + shape which each portion of the second plate 12 being located between adjacent arms or projections of the first plate 11.

For example, the sensor may be configured as an array of sub sensors (equivalent to sensor 10), in order to provide output from separate zones. Such a configuration is shown schematically in Fig. 15, including a battery 30 and controller/IC/measurement means 31.

Fig. 15A shows a second side of a sensor, and illustrates a gap 'd' between the first plate 11 and the second plate 12, such that when viewed in a projection on a plane including the second plate 12 the projection of the first plate does not overlap with the second plate.

Fig. 15B shows a first side of the sensor 10 of Fig. 15A. Fig. 15C shows a cross-section view illustrating a preferred offset through the thickness of sensor 10 between the first plate(s) 11, and the second plate(s) 12.

If present, such zone output data may be used to derive further useful information such as directionality and/or migration of moisture or wetness between/across zones. Alternatively, this could be achieved with the use of multiple sensors.

Fig. 16 schematically illustrates yet another alternative configuration of sensor 10. In this alternative configuration, the second plate 12 tapers towards one edge of sensor 10. In a similar manner to that described above with reference to Fig. 15, this embodiment illustrates a gap 'd' such that when viewed in a plan view, there is no overlap between first plate 11, and second plate 12. Alternatively, when viewed in plan view the gap 'd' may approach zero, or alternatively still a small overlap may represent up to approximately 5% of the area of the second plate 12.

Fig.16B schematically illustrates a cross section along line AA, while Fig. 16C schematically illustrates a cross section along line BB.

The first plate 11 and/or the second plate 12 may comprise at least one angled section.

The second plate 12 may be about 50mm long, and each portion of the second plate, or the second plate may be about 15mm or about 30mm wide.

The first plate 11 may be about 50mm long, and about 5mm wide.

It is anticipated that the external shape of the sensor 10 may take a number of forms. For example, the sensor may be approximately round, elongate rectangle, elongate oval, droplet shaped and/or any other practical variant. In the most preferred embodiments, the thickness of the sensor is small in order for the sensor to generally form a flexible strip appropriate for attachment to an article.

It will be appreciated that various regions of the sensor module may vary in dimension, particularly in sections where additional electronics such as the power supply, PCB, IC controller, communications module etc may be present.

The first plate 11 may be in the same plate as the second plate 12.

The first plate 11 is vertically offset from the second plate 12. The first plate 11 may be configured to be located, in use, nearer to article 28 to which the sensor 10 is attached than the second plate 12.

The second plate 12 may be configured to be located, in use, nearer to article 28 to which the sensor 10 is attached than the first plate 11.

The sensor 10 comprises an intermediate layer 20 provided between the first plate 11 and the second plate 12.

The intermediate layer 20 is a a dielectric layer.

The intermediate layer 20 may be or comprise a polymer or a fibreglass layer, or a gas, or air or oil filled polymer pocket or any nonconductive and/or dielectric material. In some embodiments the intermediate layer 20 may be a printed circuit board (PCB).

The intermediate layer 20 may be a substrate to which the first plate and second plate are deposited, etched and/or attached.

The intermediate layer 20 may extend past an edge of the first plate 11 or second plate 12 to provide for a surface or area to which electrical components may be attached.

In some embodiments the sensor or system may comprises one or more wings to provide for a surface or area to which electrical components may be attached.

The electrical components may be housed in one or more housings. The housing may protect the electrical components from the surrounding environments.

The electrical components may be one or more of:
a micro controller and/or processor
a battery or other energy source
a comparator
an analog to digital converter
an integrated component
a resistor
a capacitor
an inductor
a switch
a diode and/or LED
a resonance crystal
an antenna
a transistor
a communications module.
a display module.

The intermediate layer 20 may be less than 25mm in thickness, or between about 0.05 and 25 mm in thickness, or between about 0.05mm and 2.5mm in thickness.

The first plate 11 may be provided on a first side 21 of the sensor. The first side 21 is configured to be located towards the article 28 in use.

The second plate 12 may be provided on a second side 22 of the sensor. The second side 22 may be configured to be located away from the article 28 in use.

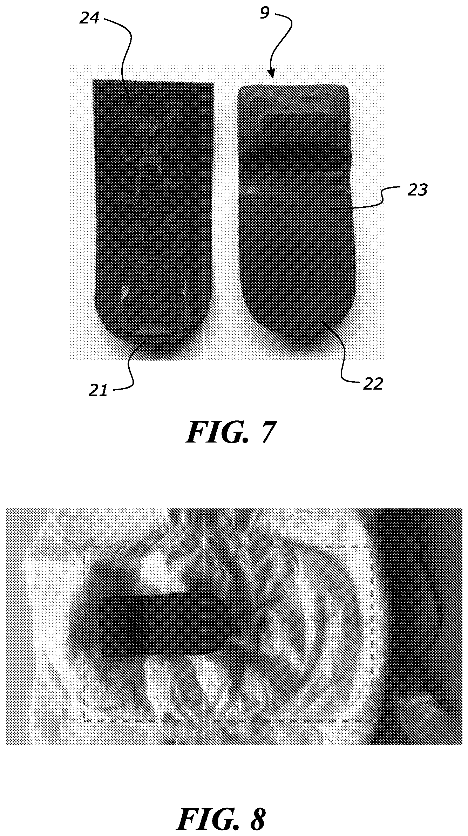

The system and/or sensor 10 may have a protective layer 23 for example as shown in Figs 7 and 8.

The protective layer 23 may be provided as an external layer configured to provide protection of the system and/or sensor.

The protective layer 23 may be configured to encapsulate the system and/or sensor.

The protective layer 23 may be a polymer layer.

The protective layer 23 may be comprise one or more of: paint, or latex paint, or rubber or glass.

The protective layer 23 may be configured to be an electrically insulating material.

The protective layer 23 may be configured to provide for a waterproof covering to stop the ingress of water into the sensor 10.

The protective layer 23 may be configured to be less than about 25mm in thickness, between about 5 and about 20 mm in thickness.

As shown for example in Fig. 7 the sensor 10 may comprise an attachment portion 24.

The attachment portion 24 may be provided on a side and/or a first side of the sensor 10.

The attachment portion 24 may be configured to allow for attachment of the sensor 10 to the article 11. Fig. 8 shows the sensor 10 affixed or attached to a nappy using by the attachment portion 24.

The attachment portion 24 may allow for connection and disconnection of the sensor 10 to and from the article 28. For example, the sensor 10 could be removed and repositioned if required, or removed and transferred to another article.

In some embodiments the sensor 10 may be reused multiple times on various articles 28.

The attachment portion 24 may comprise one or more of:
a hook and/or loop material
an adhesive
a pin or button
an electrostatic attachment mechanism.

In some embodiments, the article 28 may comprise a pocket or feature configured to retain the sensor 10.

The sensor 10 and/or system may be integrally formed with the article 28.

The sensor 10 may comprises at least one isolation layer 25.

The isolation layer 25 may be located on a side and/or the second side 22 of the sensor 10 configured to be located away from the article 28 in use.

The isolation layer 25 may comprise an insulating layer 26.

The insulating layer 26 of the isolation layer 25 may be a polymer layer. In some embodiments, the isolation layer 25 may comprise fiberglass, or glass, or a printed circuit board (PCB), an air, or gas filled pocket.

In some embodiments one or more of the first plate 11 and/or the second plate 12 may be deposited, etched and/or attached to the isolation layer 25.

The isolation layer 25 may comprise a conducting sheet 27.

The conducting sheet 27 may provide for a conductive plane.

The isolation layer 25 may be configured to isolate the sensor from the external environment.

The article may be one or more of: a nappy, a dressing (for example a wound dressing), bedding, clothing or a part of clothing, or cover or container of any kind, an object or material which is configured to take on or absorb moisture, or an object or material which is configured to dry over time (for example concrete, or wood).

In some embodiments, the article may be a wound dressing. When the wound dressing is fitted to cover a wound, it might be difficult to tell whether some liquid, such as blood or pus or other kind of body discharge, or water from outside, is there under the dressing or how much of such liquid is there.

Also disclosed is a system 9 comprising a sensor 10.

Also disclosed is a method for detecting or sensing moisture or wetness of an article to which the system is attached.

The system 9 may detect or sense moisture or wetness of an article to which the system is attached.

The system 9 may comprise a controller or processor.

The system 9 may comprise a sensor 10. The sensor 10 may be the sensor as described above.

The sensor 10 may be configured to store an electrical charge, and wherein the electrical charge storing capacity of the sensor is based on the moisture or wetness of the article.

The controller may be configured to charge the sensor for a first period 50 of time, and after the first period of time 50 discharge the sensor for a second period of time 51, and subsequently measure an output of the sensor. Preferably the output of the sensor is measured at a predetermined measurement time (e.g after discharging time). The predetermined measurement time may vary depending on the expected use of the sensor, and/or expected charge or discharge rate. In some embodiments the predetermined time may be approximately 10-1000 micro seconds after discharging.

The first period of time 50 and/or the second period of time 51 may be predetermined.

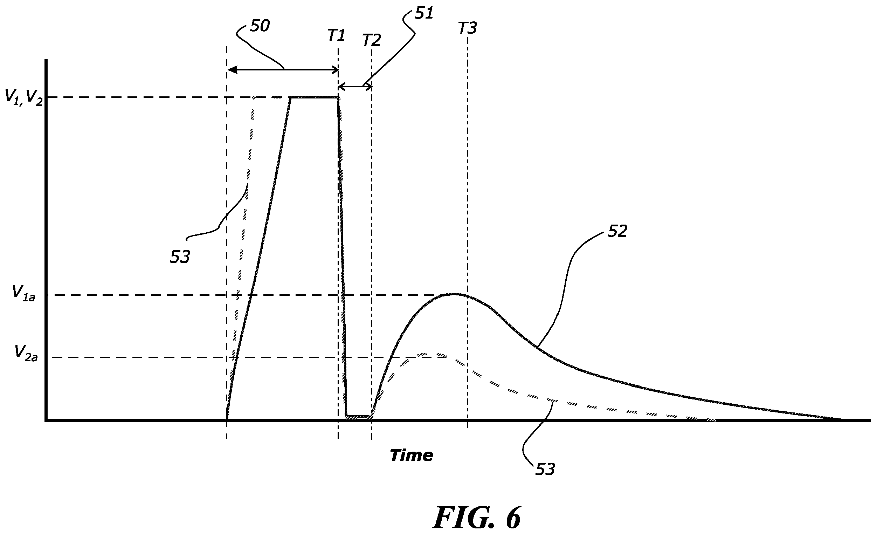

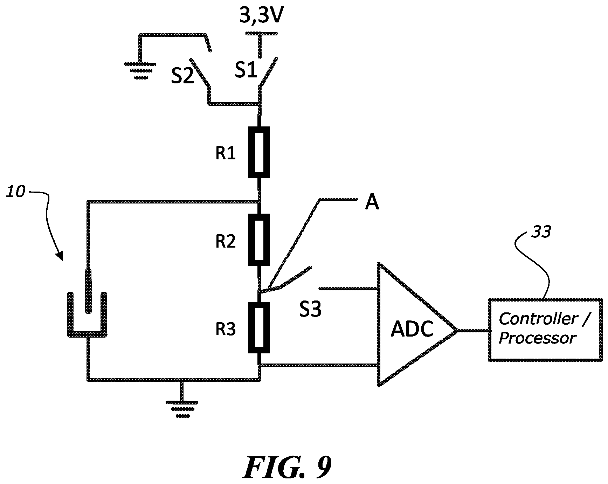

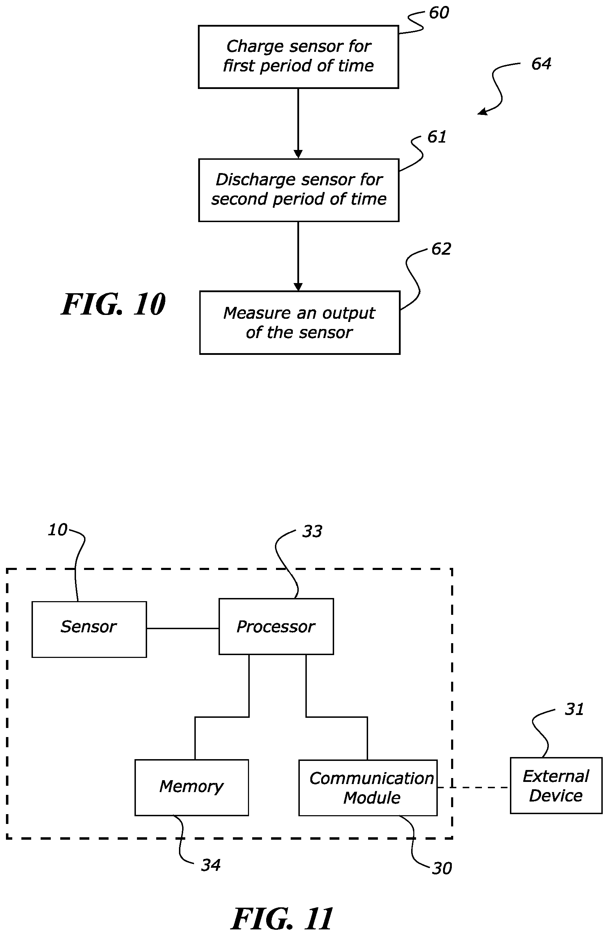

Fig. 6 shows an example of the charge and discharge of the sensor - explained in more detail below, Fig. 9 shows part of an example circuit for the system and Fig. 10 shows an example of a measurement cycle of the sensor.

The measurement cycle 64 contains a charging phase 60, a partial discharge phase 61, and a measurement phase 62.

The measurement cycle 64 may be undertaken in a sequential manner with the charging phase 60 being undertaken first followed by the partial discharge phase 61, and then the measurement phase 62.

Subsequent to the or each measurement cycle 64 the sensor 10 may be allowed to discharge to equilibrium.

The measurement cycle may be undertaken multiple times per second.

At this point an output of the sensor in this case being a voltage across the sensor 10 is V1a or V2a. In the partial discharge phase 61 the sensor 10 is discharged. In some embodiment the switch S2 is closed to introduce an additional discharge path so as to provide for a faster discharge rate. The switch S2 may be opened at the end of the discharge phase 61.

The measurement cycle starts with activating S1. This will charge the sensor (electrochemical cell like) and the parasitic capacitance of the circuit. In the charging phase 60 of the measurement cycle 64 the sensor 10 is charged for a first time period 50 to a time T1. At this point an output of the sensor in this case being a voltage across the sensor 10 is V1 or V2. The sensor 10 is charged by applying a voltage across the sensor 10 for example by closing switch S1 as shown in Fig. 9.

In the discharge or partial discharge phase 61 of the measurement cycle 64 the sensor 10 is discharged for a second period of time 51 from time T1 to time T2. E.g. the source is disconnected and S2 is activated to connect to ground for about 7µs (T2-T1). This will drain the parasitic capacitance in the circuit and invert the electrochemical cell and start the electromotive force to flow back. After this short time of starting to discharge both S1 & S2 are opened to make R1 connected to a high impedance. At the moment S2 becomes high-Z, the ionized environment keeps discharging. This discharge current or EMF will result in a voltage across R3, which is measured by the sensor, preferably at a predetermined time (T3).

When an environment with a better ability to be charged, (a wet nappy for example), more charge will build up and current will flow through R2 and R3 and thus resulting in a higher voltage measured by the ADC which correlates to the wetness of the environment.

The observed behavior is that of a small electrochemical cell. When energy is "injected", the electrolyte (air/diaper/wet diaper) is ionized. A wet diaper has a better ability to get ionized then a dry one. After the charging cycle, the cell changes from a electrolyte cell to a galvanic cell by shorting the circuit for a very small time. Due to the slowness of the cell the EMF starts to flow after this shorting of the circuit which can be measured to indicate the moisture in the environment surrounding the sensor.

In the measurement phase 62 of the measurement cycle 64 an output of the sensor is measured. The output may be based on the amount of charge and/or the amount of charge which has been discharged. In some embodiments the output of the sensor may be a based on an electrical property of the sensor. In some embodiments the output of the sensor may be a based on voltage across the sensor, or another voltage in the circuit, or a current drawn from the sensor (for example through a known resistor. In the circuit of Fig. 9 for example the output is a voltage measured at point A. In Fig. 9 the output is measured using a voltage divider circuit and an analog-to-digital converter. The output of the sensor is then provided to the processor or controller 33.

The processor or controller 33 may, based on the output of the sensor, determine an output indicative of the moisture or wetness of the article based on the measured output of the sensor 10.

As shown in Fig. 6, line 52 (broken line) shows an example measurement cycle 64 for a sensor where moisture or wetness is present, while line 53 shows an example measurement cycle 64 for a sensor where moisture or wetness is not present.

For line 52 where moisture or wetness is present at time T1 the voltage V1 the same as voltage V2 for line 53 where no moisture is present at the sensor 10. The sensor has a larger charge carrying capacity where moisture is present than where no moisture is present. Therefore, given the same charge time (the first period of time 50) and a charge time which ensures the sensor 10 will be fully charged regardless of moisture content, the sensor 10, where moisture is present will hold more charge than the sensor 10 where no moisture is present, however the voltage across the sensor will be the same (i.e. V1=V2).

Subsequently, at the end of the partial discharge phase 61 the output of the sensor 10 voltage V1a for line 52 where moisture or wetness is present will be larger than voltage V2a for line 53 where no moisture is present.

The processor or controller 33 may communicate the output indicative of the moisture or wetness of the article based to an external device 31 via communication module 30.

The communications module 30 may be a wireless communications module such as Bluetooth, Wi-Fi or other suitable RF communication module. It will be apparent that the choice of communication frequency and/or protocol may have advantages applicable to particular use scenarios. For example, relatively low frequency (800 to 900 MHz), may be advantageous in large scale environments such as hospitals, elderly homes and daycare centres where a longer wavelength can penetrate obstacles better.

Protocols such as Bluetooth (BLEv5+, Z-wave and others) may provide mesh technology to communicate over a wider area.

Alternatively, the communications module 30, may be a wired solution such as USB, ethernet, or other protocol. It will be appreciated that a wired protocol may also be used to deliver power to the sensor 10, and/or charge/recharge the sensor.

It will also be appreciated that various inductive power transfer technologies may be useful for charging/recharging an on-board battery.

The sensor 10 may comprise one or more plates. The plates may be electrically conductive. The plates may have any of the features of characteristics of the plates as described above.

The sensor 10 may be charged by providing or applying a voltage or potential difference across the sensor.

The sensor 10 may be charged by providing a voltage or potential difference across a or the first plate and a or the second plate.

The sensor may be charged with a constant voltage

The first period of time may be about 30 µs.

The first period of time may be about 20 µs, or about 10 µs, or about 5 µs, or about 2 µs.

The second period of time may be about 7 µs.

The sensor is discharged through a known resistance.

The output of the sensor is measured by an analog to digital converter and/or via a voltage divider circuit or any other suitable measuring method.

The system may comprises at least one memory element 34.

The system may comprise one or more switches (for example S1 and S2) configured to be controlled by said processor to charge and/or discharge the sensor.

The switches may be any switch known in the art for example one or more transistors.

The output of the sensor may be proportional to the wetness or moisture of the article.

The output of the sensor may be higher when the article is wet or moist than when the article is dry.

The output of the sensor may be lower when the article is dry than when the article is wet or moist.

Fig. 11 shows a diagrammatic overview of the system. The system may comprise one or more communications modules 30. The communication module 30 may be provided as part of the processor or other electrical components.

The communication module 30 may provide for communication between the system and an external device 31. The communication module 30 may provide for communication via a wired, and/or wireless connection.

The communication module 30 may be configured to provide any output of the sensor and/or system.

A display module may also be provided. The display module may display any output of the sensor and/or system.

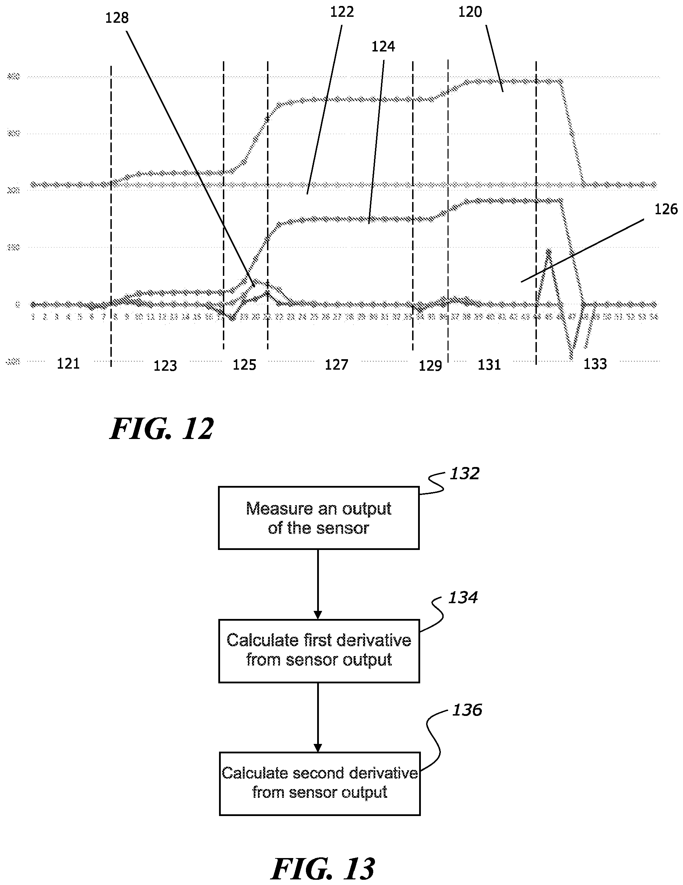

In some embodiments the output indicative of the moisture or wetness and the point of saturation of the article is determined using first and second derivates of the difference between consecutive measurement readings from the sensor, as shown in Fig. 13.

The output indicative of the moisture or wetness and the point of saturation of the article is further based on the comparison between first and second derivatives of the difference between the consecutive measurement readings from the output of the sensor.

In some embodiments, the raw data output from the sensor 10 may be processed on board the sensor.

Alternatively, the raw data from the sensor 10 may be communicated by the communications module 30 to an external device which may further process the data, by calculating for example:
- filtering the data,
- calculating a first derivative,
- calculating a second derivative
- averaging
- comparing any of the above to a predetermined set point
- any other calculation described herein.

The external device may for example be a smart phone, tablet, computer, cloud server, database server or dedicated controller. It will be appreciated that the environment in which the device is intended to be used will at least to some degree dictate preferred configurations. For example, offboard calculation and analysis of the data stream output (wired, or wirelessly) from a sensor 10, may reduce the power requirements of the central module 10 thereby prolonging the expected life from a given battery/charge.

Alternatively, in configurations where a standalone system is preferred, the sensor module 10 may incorporate additional processing steps of the data output and/or may include a user interface in order to enable alerts and/or user interaction to communicate events related to the wetness or moisture of an article etc.

Fig. 12 shows a graph detailing a series of measurement readings from the sensor over time and the corresponding first and second derivatives of these readings.

The determination of the output indicative of the moisture or wetness and the point of saturation of the article based on a comparison between the first and second derivatives will be explained further.

At each period of time, the sensor undertakes a measurement cycle as described in relation to Fig. 6. This measurement cycle is recurrent and independent of the previous cycles. Each measurement cycle produces a reading.

Preferably measurements are taken autonomously, and may occur for example periodically at a predetermined interval, or alternatively still may occur in response to a trigger such as an input from a user or external source.

Each measurement reading is taken by charging and discharging the sensor as shown in and described in relation to Fig. 6. The value obtained at T2 is used as the measurement reading.

Each value obtained at T2 is taken and stored as a measurement reading as an input for analysis. In one embodiment, a number of values are obtained at T2 for each measurement cycle. The number of readings may be averaged to determine the measurement reading for the measurement cycle. This helps to eliminate noise.

Line 120 of Fig. 12 shows an example series of measurement readings over time as described above for a sensor as it progresses through a number of stages (121, 123, 125, 127, 129, 131, 133) of varying moisture or wetness levels of the article, as will be described.

The output indicative of the moisture or wetness of the article is based on an initial base-line measurement or an earlier measurement, as shown by line 122. The initial base-line measurement in this embodiment is the sensor measurement readings taken before the sensor is placed on the article, as shown by region 121 of the graph in Fig. 12.

The base-line measurement or earlier measurement is dependent on the material used to construct the sensor.

In some embodiments, after a measurement reading is taken by the sensor, the base-line measurement or earlier measurement is then removed from this reading, as shown by the readings of line 124 in Fig. 12.

In this embodiment the controller or processor determines a first derivative of the difference between the consecutive measurement readings at each time period, as will be appreciated.

In this embodiment the controller or processor then determines a second derivative of the difference between the consecutive first derivative calculations at each time period, as will be appreciated.

Line 128 shows the series of first derivative calculations of the measurement readings shown by line 124 over time. Line 126 shows the second derivative calculations over time.

In this embodiment each first derivative and second derivative are calculated in real time as the sensor takes measurement readings.

Once calculated, the first and second derivative are compared by the controller or processor. This comparison between the first and the second derivative calculations can be used to determine when of the moisture or wetness of the article is changed and/or when the saturation point of the article has been reached.

In this embodiment the controller or processor compares the current or most up-to-date first and second derivative data points. In some embodiments, the controller or processor also compares the current or most up-to-date first derivative data point to previous first derivative data points, and the current or most up-to-date second derivative data point to previous second derivative data points.

Comparing these data points allows for the different states of varying moisture or wetness, including the saturation point, to be determined. As will be explained by way of example and with reference to Fig. 12 below, the different regions of the graph 121, 123, 125, 127, 129, 131, 133 each indicate an example of a different or varying moisture or wetness level of the sensor or article.

Firstly, no first derivative peak and no second derivative peak indicates that the sensor is in constant or unchanging state of moisture or wetness. This is shown in region 121 where the sensor has not yet been placed on the article, and region 127, where the sensor is reading a constant level of moisture or wetness.

A small first derivative peak and no or almost no second derivative peak indicates that the sensor has been attached to an article. This is shown by way of example in region 123 of Fig. 12.

A large negative first derivative peak and a second derivative peak going from positive to negative indicates removal of the sensor from an article having a high moisture or wetness level. An example of this is shown in region 133.

A positive first derivative peak and second derivative peak going from negative to positive indicates that the moisture or wetness level is increasing. This is shown in region 125 where there is a large moisture or wetness increase.

A slight positive first derivative peak and a second derivative peak going from negative to positive indicates that the article is approaching its saturation point. This is shown at region 129 of Fig. 12.

A decreasing first derivative peak and a decreasing second derivative peak following states indicating a change in moisture or wetness levels such as those described in relation to regions 123, 125 or 129 of Fig. 12 indicates that the article has reached its saturation point.

It will be appreciated that different articles will have different saturation points and the saturation point of each article will be determined by the material of the article.

In some embodiments, once the saturation point of the article has been reached, and this has been determined by the controller or processor, this will trigger a signal. This signal may alert the user to the saturation of the article.

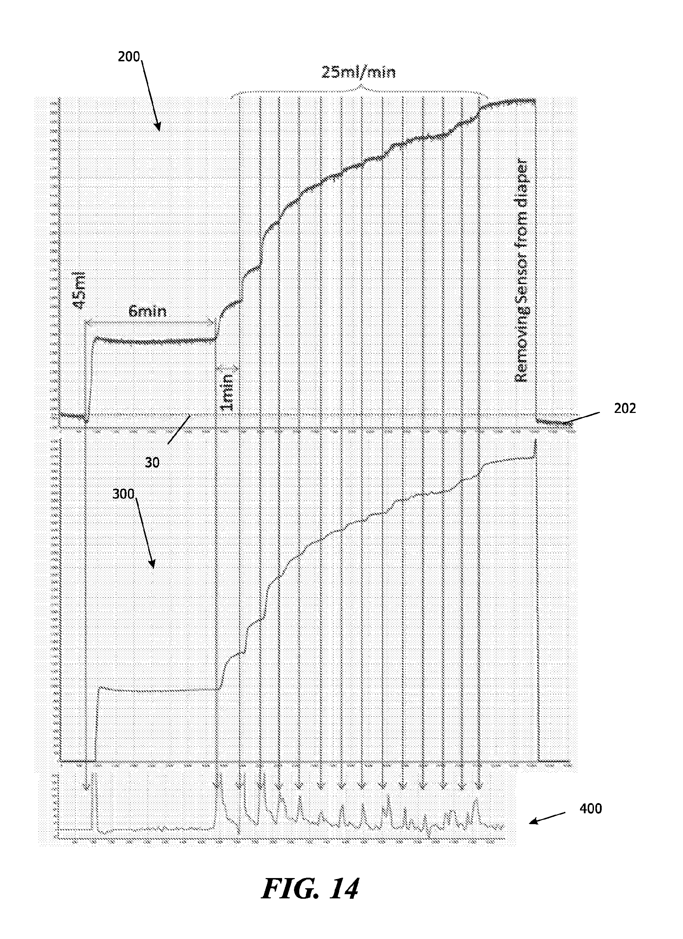

With reference to Fig. 14 test results of a sensor 10 according to the present invention will be described. In this test, a sensor 10 was attached to the exterior (dry side) of a nappy, and water was periodically injected into the inside of the nappy. In these tests, the nappy remained stationary throughout the duration of the test.

The test protocol adopted (and shown in Fig. 14) comprised:
step 1 - injecting 45 mL of water over a 14 second time period
step 2 - wait 6 minutes
step 3 - inject 25 mL of water over a 7 second time period
step 4 - wait 1 minute
step 5 - repeat 25 mL injection over a 7 second time period until leakage occurs.

With reference to series 200 shown in Fig. 14, the raw sensor output data is shown. Broken line 201 represents the baseline (with sensor 10 attached to the nappy) before any water is added to the nappy. It can be seen that the final part of the plot 202, drops below the original baseline because the sensor was removed from the nappy.

With reference to series 300 shown in Fig.14, a filtered plot of the raw data is shown.

The first portion of the plot shows 45 mL of water injected over a 14 second time period. The plot then stabilises for a time period of 6 minutes corresponding to the 6 minute wait, where no further moisture was added.

Subsequent to the 6 minute wait period, a series of one-minute steps are shown within which 25 mL of water were injected.

In the final portion of the plot, the nappy becomes saturated and further injections of water are masked in the sensor output as leakage occurred.

It can be seen from analysing the series 200 and series 300 plots that there is a strong correlation between the volume of water injected and the sensor output sensed by sensor 10. This strong correlation is particularly useful given that the sensor 10 is a non-contact type sensor and was attached to the exterior (dry side) of the nappy.

Further, with reference to series 400 as shown in Fig. 14 a plot of the first derivative of the filtered data is shown. From this plot peaks corresponding to the injection of liquid into the nappy can clearly be seen. Accordingly, the first derivative data can be used to very strongly correlate with and identify an injection event occurring.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to."

Reference to any prior art in this specification is not, and should not be taken as, an acknowledgement or any form of suggestion that that prior art forms part of the common general knowledge in the field of endeavour in any country in the world.

Certain features, aspects and advantages of some configurations of the present disclosure have been described with reference to use of the gas humidification system with a respiratory therapy system. However, certain features, aspects and advantages of the use of the gas humidification system as described may be advantageously be used with other therapeutic or non-therapeutic systems requiring the humidification of gases. Certain features, aspects and advantages of the methods and apparatus of the present disclosure may be equally applied to usage with other systems.

## Claims

1. A sensor for detecting or sensing moisture or wetness of an article (28) to which the sensor is attached, the sensor comprising:
a first plate (11) and a second plate (12), the first plate and the second plate being electrically conductive; and
a measurement means configured for measuring a value representative for an electrical charge influenced by moisture in the vicinity of the first plate **characterized in that**:
the first plate is separated and vertically offset from the second plate by a dielectric intermediate layer (20), and
when seen in a projection on the plane of the second plate, one of the following conditions is fulfilled:
the projection of the first plate on the plane does not overlap with the second plate; or
the projection of the first plate on the plane overlaps over less than 5% of the surface area of the second plate with the second plate.

2. The sensor (10) according to claim 1, wherein when seen in a projection on the plane of the second plate, the shape of the projection of the first plate on the plane is substantially complementary with the shape of the second plate.

3. The sensor (10) of claim 1 or 2, seen in a projection on the plane of the second plate, the projection of the first plate on the plane is at a distance (d) of the second plate.

4. The sensor (10) of any one of claims 1 to 3, wherein a portion of the second plate (12) is located on each side of the first plate (11).

5. The sensor (10) of any one of claims 1 to 4, wherein the second plate (12) comprises one or more further portions, located at or near one or more edges of the first plate (11).

6. The sensor of any one of claims 1 to 5, wherein the first plate (11) comprises an elongate strip.

7. The sensor (10) of any one of claims 1 to 6, wherein the second plate (12) comprises a pair of elongate strips, each elongate strip of the pair of elongate strips being one of the first portion (14) or the second portion (15) of the second plate (12).

8. The sensor of any one of the preceding claims, wherein the intermediate dielectric layer (20) is a substrate to which the first plate and second plate are attached and wherein the intermediate dielectric layer extends past an edge of the first plate and/or the second plate to provide for a mounting surface, wherein the measurement means are provided on the mounting surface.

9. The sensor of any one of the preceding claims, wherein the measurement means are configured to charge a capacitor formed by the first and the second plate and the intermediate dielectric layer, to actively discharge said capacitor, and to measure an electric signal, preferably a voltage, between the first and the second plate after said actively discharging.

10. The sensor (10) of any one of the preceding claims , wherein the intermediate layer (20) is less than 25mm in thickness, or between about 0.05 and 25mm in thickness, or between about 0.02mm and 2.5mm in thickness.

11. The sensor (10) of any one of the preceding claims 1, wherein the sensor (10) has a protective layer (23).

12. The sensor (10) of any one of the preceding claims, wherein the sensor (10) is integrally formed with the article (28).

13. The sensor (10) of any one of the preceding claims, wherein the sensor (10) comprises a least one isolation layer (25) located on a side of the sensor (10) configured to be located away from the article (28) in use.

14. The sensor (10) of any one of the preceding claims, wherein the article (28) is one or more of:
a nappy
a dressing or a wound dressing
bedding
clothing
a cover or container
an object or material which is configured to take on or absorb moisture an object or material which is configured to dry over time.

## Patentansprüche

1. Sensor zum Detektieren oder Abfühlen von Feuchtigkeit oder Nässe eines Artikels (28), an dem der Sensor angebracht ist, wobei der Sensor Folgendes umfasst:
eine erste Platte (11) und eine zweite Platte (12), wobei die erste Platte und die zweite Platte elektrisch leitend sind; und
ein Messmittel, das ausgelegt ist, um einen Wert zu messen, der eine elektrische Ladung angibt, die durch Feuchtigkeit in der Umgebung der ersten Platte beeinflusst wird, **dadurch gekennzeichnet, dass**:
die erste Platte durch eine dielektrische Zwischenschicht (20) von der zweiten Platte getrennt und vertikal davon versetzt ist und
eine der folgenden Bedingungen erfüllt ist, wenn als eine Projektion auf der Ebene der zweiten Platte betrachtet:
die Projektion der ersten Platte auf der Ebene überlagert nicht die zweite Platte; oder
die Projektion der ersten Platte auf der Ebene überlagert weniger als 5 % der Oberfläche der zweiten Platte mit der zweiten Platte.

2. Sensor (10) nach Anspruch 1, wobei die Form der Projektion der ersten Platte auf der Ebene im Wesentlichen komplementär zu der Form der zweiten Platte ist, wenn als eine Projektion auf der Ebene der zweiten Platte betrachtet.

3. Sensor (10) nach Anspruch 1 oder 2, wobei die Projektion der ersten Platte auf der Ebene in einem Abstand (d) von der zweiten Platte erzeugt wird, wenn als Projektion auf der Ebene der zweiten Platte betrachtet.

4. Sensor (10) nach einem der Ansprüche 1 bis 3, wobei ein Abschnitt der zweiten Platte (12) auf jeder Seite der ersten Platte (11) angeordnet ist.

5. Sensor (10) nach einem der Ansprüche 1 bis 4, wobei die zweite Platte (12) einen oder mehrere weitere Abschnitte umfasst, die sich an einem oder mehreren Rändern der ersten Platte (11) oder in der Nähe davon befinden.

6. Sensor nach einem der Ansprüche 1 bis 5, wobei die erste Platte (11) einen länglichen Streifen umfasst.

7. Sensor (10) nach einem der Ansprüche 1 bis 6, wobei die zweite Platte (12) ein Paar von länglichen Streifen umfasst, wobei jeder längliche Streifen des Paars von länglichen Streifen einer des ersten Abschnitts (14) oder des zweiten Abschnitts (15) der zweiten Platte (12) ist.

8. Sensor nach einem der vorangegangenen Ansprüche, wobei die dielektrische Zwischenschicht (20) ein Substrat ist, an dem die erste Platte und die zweite Platte angebracht sind, und wobei sich die dielektrische Zwischenschicht über einen Rand der ersten Platte und/oder der zweiten Platte hinaus erstreckt, um eine Befestigungsoberfläche bereitzustellen, wobei das Messmittel auf der Befestigungsoberfläche bereitgestellt ist.

9. Sensor nach einem der vorangegangenen Ansprüche, wobei das Messmittel ausgelegt ist, um einen Kondensator, der durch die erste und die zweite Platte und die dielektrische Zwischenschicht ausgebildet wird, zu laden, um den Kondensator aktiv zu entladen und um ein elektrisches Signal, vorzugsweise eine Spannung, zwischen der ersten und der zweiten Platte nach dem aktiven Entladen zu messen.

10. Sensor (10) nach einem der vorangegangenen Ansprüche, wobei die Zwischenschicht (20) eine Dicke von weniger als 25 mm oder zwischen etwa 0,05 und 25 mm oder zwischen etwa 0,02 mm und 2,5 mm aufweist.

11. Sensor (10) nach einem der vorangegangenen Ansprüche 1, wobei der Sensor (10) eine Schutzschicht (23) aufweist.

12. Sensor (10) nach einem der vorangegangenen Ansprüche, wobei der Sensor (10) einstückig mit dem Artikel (28) ausgebildet ist.

13. Sensor (10) nach einem der vorangegangenen Ansprüche, wobei der Sensor (10) zumindest eine Isolationsschicht (25) umfasst, die auf einer Seite des Sensors (10) angeordnet ist, die ausgelegt ist, um in Gebrauch von dem Artikel (28) abgewandt angeordnet zu sein.

14. Sensor (10) nach einem der vorangegangenen Ansprüche, wobei der Artikel (28) einer oder mehrere aus Folgenden ist:
eine Windel,
ein Verband oder ein Wundverband,
Bettwäsche,
Kleidung,
eine Abdeckung oder ein Behälter,
ein Gegenstand oder ein Material, der/das ausgelegt ist, um Feuchtigkeit aufzunehmen oder zu absorbieren,
ein Gegenstand oder ein Material, der/das ausgelegt ist, um mit der Zeit zu trocknen.

## Revendications

1. Capteur pour détecter ou mesurer l'humidité ou l'état mouillé d'un article (28) auquel le capteur est fixé, le capteur (9) comprenant :
une première plaque (11) et une seconde plaque (12), la première plaque et la seconde plaque étant électriquement conductrices ; et
des moyens de mesure configurés pour mesurer une valeur représentative d'une charge électrique influencée par l'humidité à proximité de la première plaque, **caractérisé en ce que** :
la première plaque est séparée et décalée verticalement de la seconde plaque par une couche intermédiaire diélectrique (20), et
lorsqu'elle est vue dans une projection sur le plan de la seconde plaque, l'une des conditions suivantes est remplie :
la projection de la première plaque sur le plan ne chevauche pas la seconde plaque ; ou
la projection de la première plaque sur le plan chevauche moins de 5 % de l'aire de surface de la seconde plaque avec la seconde plaque.

2. Capteur (10) selon la revendication 1, dans lequel, lorsqu'elle est vue dans une projection sur le plan de la seconde plaque, la forme de la projection de la première plaque sur le plan est sensiblement complémentaire de la forme de la seconde plaque.

3. Capteur (10) selon la revendication 1 ou 2, dans lequel, lorsqu'elle est vue dans une projection sur le plan de la seconde plaque, la projection de la première plaque sur le plan est à une distance (d) de la seconde plaque.

4. Capteur (10) selon l'une quelconque des revendication 1 à 3, dans lequel une partie de la seconde plaque (12) est située de chaque côté de la première plaque (11).

5. Capteur (10) selon l'une quelconque des revendications 1 à 4, dans lequel la seconde plaque (12) comprend une ou plusieurs parties supplémentaires, situées au niveau ou à proximité d'un ou plusieurs bords de la première plaque (11).

6. Capteur selon l'une quelconque des revendications 1 à 5, dans lequel la première plaque (11) comprend une bande allongée.

7. Capteur (10) selon l'une quelconque des revendications 1 à 6, dans lequel la seconde plaque (12) comprend une paire de bandes allongées, chaque bande allongée de la paire de bandes allongées étant l'une de la première partie (14) ou de la seconde partie (15) de la seconde plaque (12).

8. Capteur selon l'une quelconque des revendications précédentes, dans lequel la couche diélectrique intermédiaire (20) est un substrat auquel la première plaque et la seconde plaque sont fixées, et dans lequel la couche diélectrique intermédiaire s'étend au-delà d'un bord de la première plaque et/ou de la seconde plaque pour fournir une surface de montage, dans lequel les moyens de mesure sont fournis sur la surface de montage.

9. Capteur selon l'une quelconque des revendications précédentes, dans lequel les moyens de mesure sont configurés pour charger un condensateur formé par la première et la seconde plaque et la couche diélectrique intermédiaire, pour décharger activement ledit condensateur, et pour mesurer un signal électrique, de préférence une tension, entre la première et la seconde plaque après ladite décharge active.

10. Capteur (10) selon l'une quelconque des revendications précédentes, dans lequel la couche intermédiaire (20) présente une épaisseur inférieure à 25 mm, ou une épaisseur comprise entre environ 0,05 et 25 mm, ou une épaisseur comprise entre environ 0,02 mm et 2,5 mm.

11. Capteur (10) selon l'une quelconque des revendications précédentes 1, dans lequel le capteur (10) présente une couche de protection (23).

12. Capteur (10) selon l'une quelconque des revendications précédentes, dans lequel le capteur (10) est formé d'un seul tenant avec l'article (28).

13. Capteur (10) selon l'une quelconque des revendications précédentes, dans lequel le capteur (10) comprend au moins une couche d'isolation (25) située sur un côté du capteur (10), configurée pour être située à l'écart de l'article (28) en utilisation.

14. Capteur (10) selon l'une quelconque des revendications précédentes, dans lequel l'article (28) est un plusieurs parmi :
une couche ;
un pansement ou un pansement de plaie ;
une literie ;
un vêtement ;
un couvercle ou un récipient ;
un objet ou un matériau qui est configuré pour prendre ou absorber l'humidité ;
un objet ou un matériau qui est configuré pour sécher au fil du temps.
